# EUROPEAN PATENT APPLICATION

(11) **EP 2 210 882 A1**
(43) Date of publication of application: **28.07.2010**
(21) Application number: 09305040.9
(22) Date of filing: 16.01.2009
(51) Int. Cl.: C07D 249/04

(54) **Synthesis of new protected azahistidine, their processes and their use in synthesises**

(71) Applicant: Commissariat à l'Énergie Atomique et aux Énergies Alternatives, 75015 Paris (FR)
(72) Inventor: Cintrat, Jean-Christophe, 91430 Igny (FR); Rousseau, Bernard, 92300 Levallois-Perret (FR)
(74) Representative: Ahner, Francis

(57) **Abstract**

The synthesis of various protected azahistidine derivatives are obtained *via* 1,3-dipolar cycloaddition reactions. The newly obtained amino acids can in particular be selectively deprotected either at the side chain or at the *N*-terminus of the amino acid and should thus allow the use of these derivatives in (solid phase) peptide synthesis

## Description

The present invention relates to new protected azahistidines (azaHis) to the processes for their preparation, to their use in organic synthesis and in particular peptide synthesis.

Unnatural α-amino acids are key components for recent developments in peptides or proteins research. These synthetic compounds have been incorporated in biologically active peptides mostly to enhance proteolytic stability or to confer peculiar properties to these peptides. In addition, conformational flexibility, selectivity, pharmacokinetics and bioavailability can also be tuned based on well-designed amino acids. In the field of nanotechnology, there is also a growing interest in synthetic amino acids that can present specific properties (metal binding ability, polymerisation precursors, electron transfer...).

Among the natural amino acids, histidine is often strongly involved in the chemistry of enzymes due to the ability of the imidazole side chain to take part in acid-base catalysis. Indeed, histidine can act both as a donor or an acceptor of protons: developing analogues could pave the way to new applications. One of the most obvious parent compounds is azahistidine where the imidazole ring has been replaced by a 1,2,3-triazole. At this point two molecules can be distinguished as being azahistidines, depending on the position of the unsaturation and consequently of the position of the hydrogen of N-H in the side chain: azahistidine **I**', called azahistidine-(1,4) and azahistidine **I**", called azahistidine-(1,5):

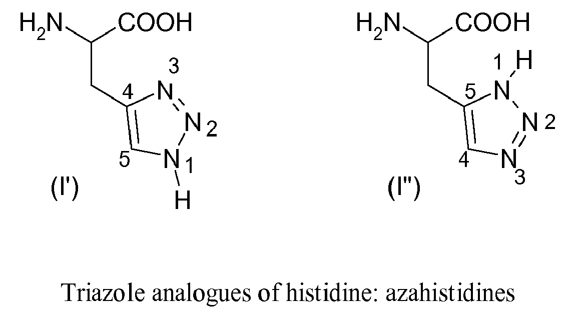

The nitrogen of the NH₂ groups, as shown in the above molecules and if need be in other amino acids, will be referred to the "N-α" nitrogens when described in a single amino acid, and to "N terminal" extremity or nitrogen when described in a peptide chain.

The carbon of the COOH groups as shown in the above molecules and if need be in other amino acids, will be referred to the "C-α-carboxyl" carbon when described in a single amino acid, and to "C terminal" extremity or carbon when described in a peptide chain.

The carbon directly linked to the N-α and to the C-α-carboxyl as shown in the above molecules and if need be in other amino acids, will be referred to the "C-α" carbon.

The chain defined by the direct succession of N-α, C-α, C-α-carboxyl will be called the "primary chain" of the amino acid, as shown in the above molecules and if need be in other amino acids.

The succession of these primary chains, linked by amide bonds also called peptide bonds, between the N-α and C-α-carboxyl of two amino acids, will be called the "backbone" of the peptide.
The rest of the molecule i.e. comprising the 1,2,3-triazole group, as shown in the above molecule will be called the "side chain".

Although the preparation of such a histidine surrogates (**I**') and (**I**") have been already described (Losikina VI, Sokolov VI (1991) Doklady Akademii Nauk SSSR 320:339-342), to the best of our knowledge, the synthetic routes published to date to obtain the fully deprotected amino acids are usually rather long (7 steps, Ikeda Y, Kawahara S-I, Taki M, Kuno A, Hasegawa T, Taira K (2003) Protein Engineering 16 : 699-706).

Recent applications based on analogues (**I**') and (**I**") include the synthesis of ^{99m}Tc radiolabelled amino acids as well as metal chelating compounds (Mindt TL, Struthers H, Brans L, Anguelov T, Schweinsberg C, Maes V, Tourwé D, Schibli R (2006) J Am Chem Soc 128 :15096-15097; Mindt TL, Muller C, Melis M, de Jong M, Schibli R (2008) Bioconjugate Chem 19 :1689-1695) It has also been demonstrated that histidine-auxotrophic bacteria (E. Coli), i.e. bacteria that can not synthesize histidine, can be supplemented with compound (**I**') or (**I**") leading to *in vivo* incorporation of this amino-acid in proteins (Ikeda Y, Kawahara S-I, Taki M, Kuno A, Hasegawa T, Taira K (2003) Protein Engineering 16 : 699-706)

However, other means than using biological organisms or biomolecules such as enzymes, to produce peptides or small proteins in a flexible way, are well known to the man skilled in the art. Solid phase peptide synthesis for example, has a particular success due to its ease of use and flexibility, enabling to obtain natural and unnatural molecules very quickly.

The triazole analogues of histidine (**I**') and (**I**"), also called azahistidines, can not be used as such in chemical peptide synthesis (solid phase particularly). These azahistidines need to be adapted in such a way for example, that the side chain groups and N-α, present little if at all reactivity to enable the C-α-carboxyl to react with the N terminal extremity only of a given peptide, or to one of its side chains only, or directly to the resin, etc. To enable this, it is critical to have protecting groups on the side chain, N-α or C-α-carboxyl according to whatever is whished for the synthesis. The same complies for more general solution phase organic chemistry using this compound. Although there have been some routes to protected (**I**') or (**I**") or derivatives, none of them deals with analogues presenting two protecting groups, especially on the N-α and on the side chain. In order for these analogues to be used in peptide synthesis it is critical to have an access to the orthogonally protected analogues.

However, no derivative of azahistidine (**I**') or (**I**") found in the literature can easily be used in a typical solid phase peptide synthesis (i.e. at the most one step for coupling and one step per deprotection). In order to efficiently use such compounds in (solid phase) peptide synthesis, access to orthogonally-protected derivatives is a necessity.

In WO 2005/021514A1, a compound presenting one protecting group on the N-α is described. Admittedly, it presents one Boc group on the N-α, but no protecting group on the side chain. A few drawbacks are noticed with this method. First, this method is not enantioselective as racemisation occurs in the process. One also has to bear in mind that this is a 6 steps synthesis to obtain the N-α mono-boc protected azahistidine. For this reason the applicant looked for a faster way to synthesise azahistidines.

T. L. Mindt et al, describe a one step synthesis of a mono-substituted azahistidine using a copper catalyst in water (Mindt TL, Struthers H, Brans L, Anguelov T, Schweinsberg C, Maes V, Tourwé D, Schibli R (2006) J Am Chem Soc 128 :15096-15097). Indeed, the recent development of Huisgen cycloaddition, so called click chemistry, which has been described using ruthenium based catalysts ( a) Zhang L, Chen X, Xue P, Sun HHY, Williams ID, Sharpless KB, Fokin VV, Jia G, (2005) J Am Chem Soc 127:15998-15999 b) Oppilliart S, Mousseau G, Zhang L, Jia G, Thuéry P, Rousseau B, Cintrat J-C (2007) Tetrahedron 63:8094-8098) or copper catalysts allows the rapid access to 1,2,3-triazoles ( a) Kolb HC, Finn MG, Sharpless KB (2001) Angew Chem Int Ed Engl, 40:2004-2021, For recent reviews see : b)Lipshutz, BH, Taft BR (2006) Angew Chem Int Ed 45:8235-8238 c) Bock VD, Hiemstra H, Van Maarseveen JH (2006) Eur J Org Chem 51-68). However using this approach, azahistidine derivatives have been obtained but no real attention has been paid to the removal of the protecting groups. The removal of such protecting groups can reveal itself quite tricky especially when said protecting groups are one or more benzyl groups.

The applicant discovered a new way to obtain fully-protected azahistidines, suitable for organic synthesis and in particular for peptide synthesis. Said azahistidines, obtained in a one step synthesis based on "click chemistry", comprise at least two protecting groups Some deprotection steps are described enabling the use of such fully-protected azahistidines in peptide synthesis. Using different catalysts the applicant was able to obtain either azahistidine-(1,4), or azahistidine-(1,5) specifically. At last, the applicant also used a protected azahistidine to synthesize a peptide of biological interest: a GHK analogue.

This invention relates to compounds of formula (**Ia**) and (**Ib**): 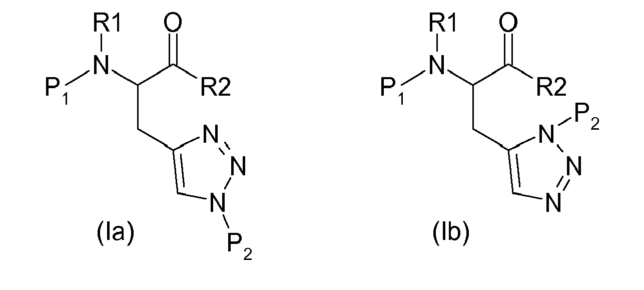 wherein
R1 is chosen between hydrogen, C₁-C₆alkyl; aryl-C₁-C₆alkyl-;
R1 is most preferably hydrogen
R2 is chosen between hydroxy, C₁-C₆alkyloxy, aryl-C₁-C₆alkyloxy;
R2 is most preferably hydroxy or ethoxy.

The term "C₁-C₆alkyl" as used in the present invention refers to straight or branched chain substituted or unsubstituted alkyl radicals containing from 1 to 6 carbon atoms including, but not limited to, methyl, ethyl, n-propyl, iso-propyl, n-butyl, isobutyl, sec-butyl, t-butyl, n-pentyl, n-hexyl and the like.

The term "aryl" as used in the present invention refers to a monocyclic or bicyclic carbocyclic ring system having one or more aromatic rings including, but not limited to, phenyl, naphthyl, tetrahydronaphthyl, indanyl and the like.

The term "C₁-C₆alkyloxy" as used in the present invention refers to any -O-R radical, wherein R is a C₁-C₆alkyl as defined above including, methyl, ethyl, propyl, tert-butyl, etc.

P1 and P2 in compounds of formula (Ia) or (Ib) are either identical or different nitrogen protecting groups.

A nitrogen protecting group is a chemical moiety linked to the nitrogen, stopping the nitrogen to react whilst the protecting group is on it, ideally said protecting group is selectively removed, or "cleaved", from the nitrogen by using specific and selective conditions.

In the present invention P1 and P2 are chosen between Fmoc, POM, ethoxycarbonyl, Boc, Bn, Bz, CBz, allyl, Alloc, Ac, formyl, DMB, PMB or all other protecting groups used in organic chemistry.
P1 is most preferably the Boc or Fmoc or Ac group.
P2 is most preferably the POM group.
Fmoc group refers to 9-Fluorenylmethyloxycarbonyl
POM group refers to pivaloyl oxymethyl, the nitrogen to be protected being linked to the CH₂- in the following structure: 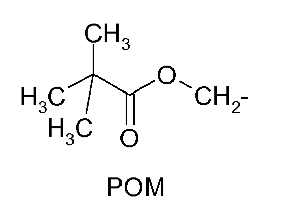 Boc (also called t-Boc or ^{t}Boc) group refers to (tert-butoxy)carbonyl
Bn group refers to benzyl
Bz group refers to benzamide
CBz (also called Z) group refers to benzyloxycarbonyl
Alloc group refers to allyloxycarbonyl
Ac group refers to acetyl
DMB group refers to 3,5-dimethoxybenzyl
PMB group refers to 4-methoxybenzyl

Peptide synthesis is a particular type of organic synthesis which relates to peptides, i.e. polymers of amino acids. An amino acid is an organic molecule comprising at least one amine group and at least one carboxylic acid group.

When, Merrifield invented solid phase peptide synthesis (SPPS) in 1963, it was according to the Boc method. To remove Boc from a growing peptide chain, acidic conditions are used (usually neat TFA). Removal of side-chain protecting groups and the peptide from the resin at the end of the synthesis is achieved by incubating in hydrofluoric acid (which can be dangerous or even deadly), although generally safe, and using only small quantities, HF cleavage needs to be done using specialized equipment, so it is generally disfavored. However for complex syntheses Boc is favourable. When synthesizing nonnatural peptide analogs which are base-sensitive (such as depsipeptides), Boc is necessary. Depsipeptides are peptides in which a peptide bond, or amide bond, in the backbone of said peptide, has been replaced by an ester bond.

In contrast to the Boc strategy SPPS described above, the Fmoc strategy SPPS uses basic conditions (usually 20% piperidine in DMF) to remove the Fmoc protecting group from the growing peptide chain. This method was introduced by Carpino in 1972 and further applied by Atherton in 1978. It was first described as a protecting group by Carpino in 1970. Removal of the peptide from the resin is usually achieved by incubating in trifluoroacetic acid (TFA), deionized water, and triisopropylsilane. These conditions often remove the side-chain protecting groups also. The main advantage of Fmoc chemistry is that no hydrofluoric acid is needed. It is therefore used for most routine synthesis.

Other protecting group strategies can be used of course, but Fmoc solid phase peptide synthesis strategy (SPPS) and Boc solid phase peptide synthesis (SPPS) strategy are preferred because of their practical aspects and ease of realisation.

With the above azahistidines (Ia) or (Ib) in hand, the next step was to selectively remove the protecting group.

Preferably, the removal of the protecting groups P₁ and/or P₂ is achieved by using soft conditions.

The azahistidines (Ia) or (Ib) described in the present invention can be used in Fmoc or Boc solid phase peptide synthesis (SPPS).

P1 and P2 can be sensitive to basic conditions. Protecting groups sensitive to basic conditions are for example Fmoc and POM.

As previously mentioned, one protecting group of interest in peptide chemistry is the Fmoc group, which is base sensitive. Thus organic or inorganic bases may be used. Suitable organic bases include amines, either primary, secondary or tertiary. Examples of suitable amines include cyclohexylamine, 1,5-diazobicyclo-[5,4,0]-undec-5ene, piperidine, ethanolamine, pyrrolidine, diethylamine, morpholine, piperazine, dicyclohexylamine, hydroxylamine, hydrazine, N,N'-isopropylethylamine, N,N,N',N'-tetramethyl-1,8-naphtalenediamine, tributylamine, triethylamine and triethylendiamine.

Inorganic bases include sodium hydroxide, potassium hydroxide and ammonia. The inorganic bases are preferably employed in conjunction with one or more organic solvents. Thus sodium hydroxide is used typically in the form of Tesser's base, i.e., dioxane-methanol-4N sodium hydroxide and ammonia as a 5 molar solution in methanol-methylene chloride. Lower concentrations of sodium hydroxide or potassium hydroxide can be sufficient to deprotect the Fmoc group. Each base may of course interact with the Fmoc in a varying manner. Thus most efficient cleavage (<15 min) is obtained with piperidine, cyclohexylamine, 1,5-diazabicyclo-[5,4,0]-undec-5-ene, ethanolamine, pyrrolidine or tesser's base. Piperidine is a particularly preferred base for cleavage of the Fmoc group.

The cleavage reaction is carried out in an organic solvent conventionally employed in solid phase peptide synthesis. Suitable solvents include the halogenated C₁-C₆alkyls, such as methylene chloride. Room temperature is most conveniently employed for the cleavage reaction.

Derivatives bearing a POM group on the triazole ring appear to be good candidates for SPPS since selective deprotection of either the side chain or the amino-acid nitrogen group proved to be possible. Azahistidine bearing a Fmoc group as P1 and a POM group as P2 is particulary interesting since the deprotection of the Fmoc group takes place with piperidine while the POM deprotection requires more basic conditions thus allowing the use of compound in SPPS as exemplified in the synthesis of a GHK analogue described herein (example 3).

The deprotection conditions used to remove POM in the present invention are KOH_{aq} in methanol or NaOH_{aq} in MeOH or NaOH_{aq} in tetrahydrofurane (THF) or KOH_{aq} in tetrahydrofurane (THF). The concentrations of the NaOH_{aq} or KOH_{aq} range from 1 mole per litre of water to 5 moles per litre of water, preferably 1 mole per litre of water.

The use and the conditions of use of methanol or THF in the POM deprotection reaction are well known from the man skilled in the art.

To remove the POM group from (Ia) or (Ib), the quantity of KOH should range from 1 equivalent to 10 equivalents in moles for every equivalent of (Ia) and/or (Ib). The same treatment can be done to a peptide or another organic molecule to remove the POM group of the azahistidine moities.

To remove the POM group from (Ia) or (Ib), the quantity of NaOH should range from 1 equivalent to 10 equivalents in moles for every equivalent of (Ia) and/or (Ib). The same treatment can be done to a peptide or another organic molecule to remove the POM group of the azahistidine moities.

P1 and P2 can be sensitive to acidic conditions. Protecting groups sensitive to acidic conditions are for example Boc, ethoxycarbonyl, benzyloxycarbonyl.

As previously mentioned, one protecting group of interest in peptide chemistry is the Boc group, which is acid sensitive. Thus organic or inorganic acids may be used. Suitable organic acids include trifluoroacetic acid (TFA), acetic acid, paratoluenesulfonic acid, methanesulfonic acid and all other conditions well known from the man skilled in the art.

Suitable inorganic acids include hydrochloric acid (HCl), hydrofluoric acid (HF), hydrobromide (HBr), sulphuric acid (H₂SO₄), Lewis acids and all other conditions well known from the man skilled in the art.

To cleave a Boc group cleanly, a scavenger can be added: for example, a type of silane with deionized water act as a perfect scavenger. The silane usually used is triisopropylsilane.

The cleavage reaction can be carried out directly into the acid with scavengers like it is usually the case with TFA or in an organic solvent conventionally employed in solid phase peptide synthesis. Suitable solvents include the halogenated C₁-C₆alkyls, such as methylene chloride. Room temperature is most conveniently employed for the cleavage reaction.

In particular types of peptide chemistry strategies, other means to remove the protecting groups P1 or P2 are sometimes necessary. Azahistidine (Ia) and (Ib) can be used in such strategies: P1 and P2 can be sensitive to the treatment with palladium (0) conditions.

Protecting groups sensitive to the treatment with palladium (0) conditions, with or without hydrogen, are for example benzyl, benzoyl, benzyloxycarbonyl, allyl, allyloxycarbonyl, PMB, DMB.

Pd⁰ (Palladium (0)) treatment is obtained by usual organic chemistry techniques. The palladium used can be Pd/C (palladium on carbon), or a palladium (0) precursor like Pd(OAc)₂, in a adequate solvent, which can be chosen between ethanol, tetrahydrofurane (THF), with or without triphenylphosphine, with or without hydrogen bubbling, and with or without acid (acetic acid, TFA) depending on the protecting group to be removed. These techniques are well known from the man skilled in the art.

P1 and P2 can be sensitive to oxidative conditions. Protecting groups sensitive to the treatment with oxidative conditions, are for example PMB, DMB. Oxidative conditions can be induced by the use for example of CAN, DDQ and the conditions well known from the man skilled in the art.
CAN refers to ceric ammonium nitrate
DDQ refers to 2,3-dichloro-5,6-dicyanobenzoquinone

In particular types of peptide chemistry strategies, Azahistidine (Ia) or (Ib) with P1 and P2 being non-sensitive to all of the above listed conditions can be used. Such protecting groups can be Ac, which is actually used to block the synthesis of certain unwanted peptides during a SPPS.

This process which is called the capping process, usually uses acetic anhydride in large excess with a base like pyridine or diisopropylethylamine.

Preferably, in the present invention P1 and P2 are orthogonal and chosen between Fmoc, POM, ethoxycarbonyl, Boc, Bn, Bz, CBz, allyl, Alloc, Ac, formyl, DMB, PMB or all other protecting groups used in organic chemistry.
Orthogonal means in this case that the deprotecting conditions for P1 do not remove or affect structurally P2, or the deprotecting conditions for P2 do not remove or affect structurally P1.

An example of orthogonal groups in this particular invention can be Boc and Fmoc, or Boc and POM, or Fmoc and POM for either which of P1 and P2.

For peptide synthesis in particular, it is preferable that P₁ is either Fmoc or Boc. P₂ can then be chosen between POM, ethoxycarbonyl, Bn, Bz, CBz, allyl, Alloc, Ac, formyl, DMB, PMB.

More preferably in compound (Ia), R1 is hydrogen, R2 is hydroxy, the protecting group P₁ is chosen between Fmoc and Boc, and the protective group P₂ is POM.

The final protected azahistidines (Ia) and/or (Ib) can be unnatural amino acids of the L series, in which case all the components of the synthesis are chosen in consequence. Indeed, the configuration of the C-α carbon and enantiomeric purity, or excess of the azahistidines, are the same as the starting material, which is commercial.

The final protected azahistidines (Ia) and/or (Ib) can be unnatural amino acids of the D series, in which case all the components of the synthesis are chosen in consequence. Indeed, the configuration of the C-α carbon and enantiomeric purity, or excess of the azahistidines, are the same as the starting material, which is commercial.

Depending on the choice of the catalyst and the solvent, different regioisomers can be obtained: azahistidines (Ia) were obtained using the copper-catalysed click chemistry. The more recently described ruthenium-catalysed cycloaddition, afforded the azahistidines (Ib). Using either condition, rapid access to protected azahistidines was demonstrated and the regioisomers were obtained with fair to satisfactory yields (example 1).

To obtain the desired protected azahistidines, the following reaction is the key step of the invention: 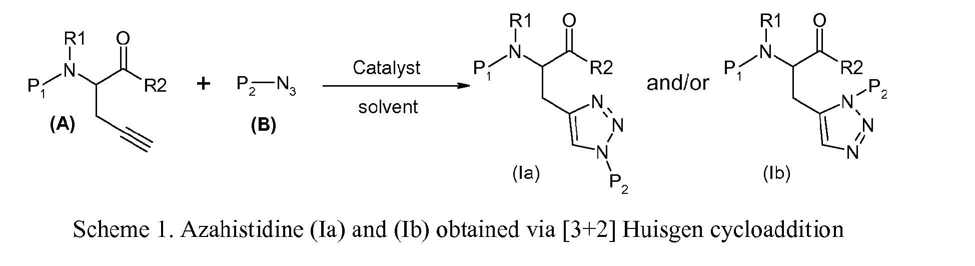 wherein
R1 is chosen between hydrogen, C₁-C₆alkyl; aryl-C₁-C₆alkyl-;
   R1 is most preferably hydrogen
R2 is chosen between hydroxy, C₁-C₆alkyloxy, aryl-C₁-C₆ alkyloxy;
   R2 is most preferably hydroxy or ethoxy.

The term "C₁-C₆alkyl" as used in the present invention refers to straight or branched chain substituted or unsubstituted alkyl radicals containing from 1 to 6 carbon atoms including, but not limited to, methyl, ethyl, n-propyl, iso-propyl, n-butyl, isobutyl, sec-butyl, t-butyl, n-pentyl, n-hexyl and the like.

The term "aryl" as used in the present invention refers to a monocyclic or bicyclic carbocyclic ring system having one or more aromatic rings including, but not limited to, phenyl, naphthyl, tetrahydronaphthyl, indanyl and the like.

The term "C₁-C₆alkyloxy" as used in the present invention refers to any -O-R radical, wherein R is a C₁-C₆alkyl as defined above including, methyl, ethyl, propyl, tert-butyl, etc.

The compound of formula (A) is called "protected propargyl amino acid" or "P1 protected propargyl amino acid" and the compound (B) is called "protected azide" or "P2 protected azide".

P1 in the compound of formula (A) is either an identical or different nitrogen protecting group to P2 in compound of formula (B).

In the present invention P1 and P2 are chosen between Fmoc, POM, ethoxycarbonyl, Boc, Bn, Bz, CBz, allyl, Alloc, Ac, formyl, DMB, PMB or all other protecting groups used in organic chemistry.
P1 is most preferably the Boc or Fmoc or Ac group.
P2 is most preferably the POM group.
Fmoc group refers to 9-Fluorenylmethyloxycarbonyl
POM group refers to pivaloyl oxymethyl, the nitrogen to be protected being linked to the CH₂- in the following structure: 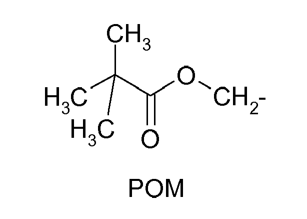 Boc (also called t-Boc or ^{t}Boc) group refers to (tert-butoxy)carbonyl
Bn group will refer to benzyl
Bz group refers to benzamide
CBz (also called Z) group refers to benzyloxycarbonyl
Alloc group refers to allyloxycarbonyl
Ac group refers to acetyl
DMB group refers to 3,5-dimethoxybenzyl
PMB group refers to 4-methoxybenzyl

The success of the method depending on P1, P2, R1, R2 and the regioisomers (Ia) or (Ib) wanted is therefore determined by the choice of :
a) a catalyst chosen between:
   copper catalysed version of Huisgen cycloaddition, wherein the copper is copper (I), preferably chosen is between: Cu(OAc)₂ with sodium ascorbate, Cu/C, CuI/DiPEA, CuI(PPh₃), CuBr(Et₃N), CuBr(2,6-lutidine), copper found in zeolites or CuSO₄ with sodium ascorbate;
   ruthenium catalysed version of Huisgen cycloaddition, wherein the ruthenium catalyst is preferably chosen between from Cp^{*}RuCl(PPh₃)_{2,} Cp*RuCI(COD) or Cp*RuCI(NBD).
   2,6-lutidine being: 2,6-dimethylpyridine
   Zeolites used being: Y, USY, MOR, ZSM5
   Cp* being: pentamethylcyclopentadienyl
   COD being: cyclooctadienyl
   NBD being: bicyclo[2.2.1]hepta-2,5-diene
b) a solvent which can be chosen from any one of the group consisting of: water, tert-butanol, acetonitrile, dimethylformamide (DMF), benzene, toluene or mixture thereof.

Preferably, the copper catalysed version of Huisgen cycloaddition, wherein the copper catalyst is Cu(OAc)₂, is used with a solvent comprising tert-butanol /water.

Preferably, the ruthenium catalysed version of Huisgen cycloaddition, wherein the ruthenium catalyst is Cp^{*}RuCl(PPh₃)_{2,} is used with a solvent comprising toluene or benzene or a mixture thereof.

A solvent is a substance that can dissolve another substance, or in which another substance is dissolved, forming a solution.

A catalyst is a substance, usually used in small amounts relative to the reactants, that modifies and increases the rate of a reaction without being consumed in the process.

To obtain the corresponding azahistidines as described in the present invention, the ratio between P1 protected propargyl amino acid and P2 protected azide should be comprised between 200 and 10 % in mass.
Preferably, to obtain the corresponding azahistidines as described in the present invention, the ratio between Fmoc protected propargyl amino acid and POM protected azide should be comprised between 200 and 10 % in mass.
Preferably, to obtain the corresponding azahistidines as described in the present invention, the ratio between Boc protected propargyl amino acid and POM protected azide should be comprised between 200 and 10 % in mass.

To obtain the corresponding azahistidines as described in the present invention, the ratio between the catalyst and P1 protected propargyl amino acid should be comprised between 1 and 50% in mass.
Preferably, to obtain the corresponding azahistidines as described in the present invention, the ratio between Cu(OAc)₂ catalyst and Fmoc protected propargyl amino acid should be comprised between 5 and 20% in mass.

Preferably, to obtain the corresponding azahistidines as described in the present invention, the ratio between Cu(OAc)₂ catalyst and Boc protected propargyl amino acid should be comprised between 2 and 20 % in mass.
Preferably, to obtain the corresponding azahistidines as described in the present invention, the ratio between Cu(OAc)₂ catalyst and sodium ascorbate should be comprised between 100 and 10 % in mass.

To obtain the corresponding azahistidines as described in the present invention, the ratio between the solvent and P1 protected propargyl amino acid should be comprised between 1000 and 50000 % in mass.

Preferably, to obtain the corresponding azahistidines as described in the present invention, the ratio between tBuOH as solvent and Boc protected propargyl amino acid should be comprised between 5000 and 20000 % in mass.

Preferably, to obtain the corresponding azahistidines as described in the present invention, the ratio between tBuOH as solvent and Fmoc protected propargyl amino acid should be comprised between 5000 and 20000 % in mass.

Preferably, to obtain the corresponding azahistidines as described in the present invention, the ratio between tBuOH as solvent and water as solvent should be comprised between 2000 and 100 % in mass.

The protected azahistidines can also be used in solution phase or solid phase organic synthesis to prepare new derivatives.

For example, it is possible to hydrolyse R2, by saponification for example, if R2 is not a hydroxy, to obtain a carboxylic acid. Said carboxylic acid is then usable in a peptide synthesis or more generally in an organic synthesis.

In organic synthesis, liquide phase peptide synthesis and solid phase peptide synthesis, the use in a synthesis of the protected azahistidine (Ia) or (Ib) can imply the removal of the protecting groups P₁ and/or P₂. Indeed, the selective removal of P₁ or P₂ enables to selectively work on one function/group of the molecule whilst the others are blocked by protecting groups.

For example, in the case of peptide synthesis, when a azahistidine (Ia) or (Ib) is positioned at the N terminal end of the chain, both protecting groups P1 and P2 can be removed at the same time. No orthogonal protecting groups are needed, though in routine the last residue can have orthogonal protecting groups even so.

When azahistidine (Ia) or(Ib) are positioned in the middle of a peptide chain, P1 and P2 of the azahistidine derivative(s) need to be orthogonal. All protecting groups of the secondary chains of the amino acids of the peptide will need to be removed either with all other protecting groups at the end of the synthesis, or selectively to work on this secondary chain specifically (oxidation, reduction, react the chain to other organic molecules, etc.), whilst the other protecting groups of the peptide are still in place.

The classical way to elongate a peptide in peptide synthesis is from the C terminal extremity towards the N terminal. Sometimes for experimental reasons, it is necessary to work specifically on the C terminus extremity. In this case, azahistidines with an ester for example or another alterable group into the Cα-carboxylic acid might be useful. The present invention also enables the access to such azahistidines (Ia) or (Ib).

The following examples further illustrate, but do not limit the invention.

### Example 1: structures of some synthesized azahistidines

Using the copper-catalyzed version of the reaction, we were able to react either Fmoc- or ^{t}Boc- protected propargylglycine with a range of azides (Entries 1-8 in Table 1) yielding the expected triazoles. It was found that the purification of the acid products was sometimes difficult resulting in low yields. When the N-acetyl, ethyl ester of propargylglycine was used as a starting material, in conjunction with ruthenium catalyst, the expected 1,5-regioisomer was also cleanly obtained (Entries 9 and 10 in Table 1). The cycloaddition starting from ^{t}Boc or Fmoc protected propargylglycine yielded the expected pivaloyloxymethyl (POM) azahistidine cleanly (Entries 4 and 8 in Table 1).

**Table 1. Synthesis of azahistidine analogues**

| Entry | Propargyl glycine derivative | Azide | Conditions | Product | Yield (%) |
|---|---|---|---|---|---|
| 1 | 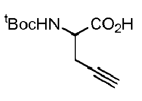 | Bn-N₃ | A | 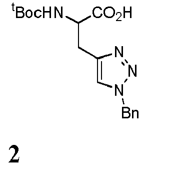 | 60 |
| 2 | | PMB-N₃ | A | 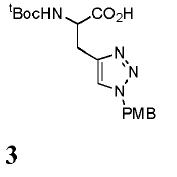 | 40 |
| 3 | | DMB-N₃ | A | 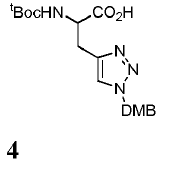 | 15 |
| 4 | | 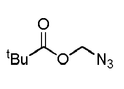 | A | 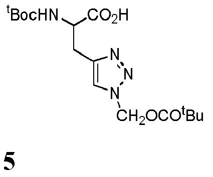 | 73 |
| 5 | 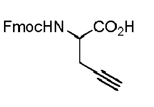 | Bn-N₃ | A | 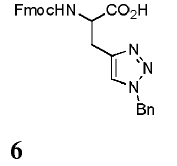 | 70 |
| 6 | | PMB-N₃ | A | 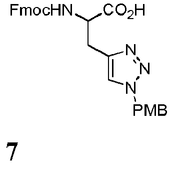 | 40 |
| 7 | | DMB-N₃ | A | 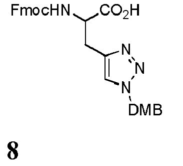 | 30 |
| 8 | | 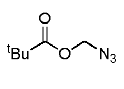 | A | 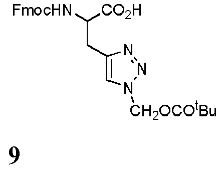 | 57 |
| 9 | 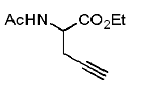 | Bn-N₃ | B | 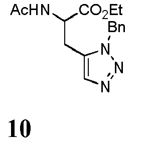 | 84 |
| 10 | | DMB-N₃ | B | 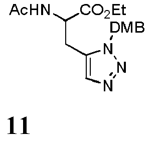 | 44 |

| | | | | | |
|---|---|---|---|---|---|
| Conditions A: Cu(OAc)₂/sodium ascorbate in ^{t}BuOH/H₂O at r.t.. B: Cp^{*}RuCl(PPh₃)₂ in toluene at 80 °C. | | | | | |

### General method (A): Synthesis of Boc-β-(1-benzyl)-1,2,3-triazol-4-yl)-Alanine 2

107 mg (0.5 mmol) of *N*-Boc-propargylglycine were introduced in a 100 mL round-bottom flask in 15 mL of ^{t}BuOH together with 70 mg (0.5 mmol, leq.) of benzylazide. 10 mg (0.05 mmol, 0.01 eq.) of Cu(OAc)₂.xH₂O dissolved in 3 mL of water and 20 mg (0.1 mmol, 0.02 eq.) of Na-ascorbate in 2 mL of water were further introduced. The mixture was stirred overnight at room temperature. ^{t}BuOH was then removed by rotary-evaporation and the pH of the resulting aqueous mixture was adjusted to 2 with a 1 M HCl solution. The aminoacid was extracted three times with ethylacetate (50 mL) and the organic phases, pooled together were washed with brine and dried over anhydrous MgSO₄. After filtration, the crude product was evaporated and dissolved in ethyl acetate. After crystallisation in cyclohexane and filtration 104.3 mg of the expected Boc-β-(1-benzyl)-1,2,3-triazol-4-yl-Alanine **2** were obtained (60 % yield).

Purification conditions for the other derivatives were as follows:
Boc-β-(1-(4-methoxybenzyl)-1,2,3-triazol-4-yl)-Alanine **3** was purified by flash chromatography using an isocratic elution consisting in DCM/MeOH/acetic acid 90:10:1. The fractions were pooled, dissolved in chloroform and crystallised from hexane.
Boc-β-(1-(3,5-dimethoxybenzyl)-1,2,3-triazol-4-yl)-Alanine **4** was obtained as a colourless oil without crystallization.
Boc-β-(1-pivaloyloxymethyl)-1,2,3-triazol-4-yl)-Alanine **5** were purified by flash chromatography using an isocratic elution consisting in DCM/MeOH/acetic acid 96:4:5 %.
Fmoc-β-((1-benzyl)-1,2,3-triazol-4-yl)-Alanine **6** was dissolved in ethyl acetate and crystallised with pentane.
Fmoc-β-(1(-4-methoxybenzyl)-1,2,3-triazol-4-yl)-Alanine **7** was dissolved in chloroform and crystallised with hexane.
Fmoc-β-(1-(3,5-dimethoxybenzyl)-1,2,3-triazol-4-yl)-Alanine **8** was dissolved in ethyl acetate and crystallised with cyclohexane.
Fmoc-β-(1-pivaloyloxymethyl)-1,2,3-triazol-4-yl)-Alanine **9** was purified by flash chromatography using an isocratic elution consisting in DCM/MeOH/acetic acid 97:3:5 %.

### General method (B): Synthesis of Ac-(1-(3,5-dimethoxybenzyl)-1,2,3-triazol-5-yl)-Ala-OEt 10

In a 50 mL two-necked round bottom flask was introduced 10 mL of anhydrous toluene. This solvent was then degassed by three vacuum freeze/thaw cycles. 100 mg (0.54 mmol) of *N*-acetylpropargylglycine ethyl ester were then introduced together with 145 mg (1.09 mmol, 2 eq.) of benzyl azide and the mixture was degassed once more. Finally 21.7 mg (27.3 µmol, 0.05 eq.) of Cp^{*}RuCl(PPh₃)₂ were introduced and the mixture was degassed once again. The reaction mixture was then heated to 80 °C under nitrogen for 15 h. The resulting mixture was then evaporated to afford 205.9 mg of crude product. This product is dissolved in acetonitrile and filtered. The liquid was further purified by preparative HPLC using a water/acetonitrile/formic acid gradient, yielding 97.12 mg of viscous oil for a yield of 44%.

### Analytical data:

### Boc-β-(1-benzyl)-1,2,3-triazol-4-yl)-Alanine 2

¹H NMR (400 MHz; CDCl₃): δ 1.27 (s, 9H, ^{t}Bu), 3.15 (br. s, 2H, C_{β}HH'), 4.32 (br. s, 1H, C_{α}H), 5.32 (d, *J* = 15.0, 1H, CH-Bzl), 5.38 (d, 1H, *J* = 15.0, CH'-Bzl), 5.97 (br. s, 1H, HNC_{α}), 7.13-7.19 (m, 2H, Bzl), 7.23-7.28 (m, 3H, Bzl), 7.32 (br. s, 1H, CH triazol).
MS (ESI): 347.1 (M+H)⁺

### Boc-β-(1-(4-methoxybenzyl)-1,2,3-triazol-4-yl)-Alanine 3

¹H NMR (400 MHz; CDCl₃): δ 1.42 (s, 9H, ^{t}Bu), 3.15-3.25 (m, 1H, C_{β}H), 3.29-3.39 (m, 1H, C_{β}H'), 3.81 (s, 3H, *p*-methoxy), 4.49 (bs, 1H, C_{α}H), 5.43 (s, 2H, CH₂-Bzl), 5.73 (br. s, 1H, HNC_{α}), 6.90 (d, *J* = 8.4, 2H, H-3,5 Bzl), 7.22 (d, *J* = 8.4 Hz, 2H, H-2,6 Bzl), 7.31 (br. s, 1H, CH triazol)
MS (ESI): 377.1 (M+H)⁺

### Boc-β-(1-(3,5-dimethoxybenzyl)-1,2,3-triazol-4-yl)-Alanine 4

¹H NMR (400 MHz; CD₃OD): δ 1.36 (s, 9H, ^{t}Bu), 3.05 (dd, *J* = 14.9, *J* = 4.5, 1H, C_{β}H), 3.25 (dd, *J* = 14.9 , *J* = 8.9, 1H, C_{β}H'), 3.74 (s, 6H, m,m-dimethoxy), 4.37 (dd, *J* = 8.9, J = 4.5, 1H, C_{α}H), 5.48 (s, 2H, CH₂-Bzl), 6.43 (bs, 3H, Bzl), 7.73 (s, 1H, CH triazol)
MS (ESI): 407.1 (M+H)⁺

### Boc-β-(1-(pivaloyloxymethyl)-1,2,3-triazol-4-yl)-Alanine 5

¹H NMR (400 MHz; d6-DMSO): δ 1.11 (s, 9H, ^{t}Bu), 1.45 (s, 9H, ^{t}Bu), 2.94 (dd, 1H, *J* = 14.5, *J* = 9.7, C_{β}H), 3.09 (dd, 1H , *J* = 14.5, *J* = 4.1, C_{β}H), 4.10-4.19 (m, 1H, C_{α}H), 6.27 (s, 2H, CH₂ POM), 7.08 (d, 1H, *J* = 7.9, NH), 7.94 (s, 1H, CH triazol).
¹³C NMR {¹H} (100 MHz, d6-DMSO): δ 26.87, 27.44, 28.53, 38.60, 53.80, 70.27, 78.52, 124.45, 144.14, 155.76, 173.52, 176.84.
MS (ESI): 371.7 (M+H)⁺
IR (KBr): 3386, 3151, 2981, 2938, 2576, 1747, 1716, 1517 cm⁻¹

### Fmoc-β-((1-benzyl)-1,2,3-triazol-4-yl)-Alanine 6

¹H NMR (400 MHz; CDCl₃): δ 3.36 (dd, *J* = 14.8, *J* = 5.4, 1H, C_{β}H), 3.42 (dd, *J* = 14.8, *J* = 3.8, 1H, C_{β}H'), 4.16 (t, *J* = 6.6, 1H, CH Fmoc), 4.30-4.41 (m, 2H, CH₂ Fmoc), 4.57-4.66 (m, 1H, C_{α}H), 5.37 (d, *J* = 14.9, 1H), 5.51 (d, *J* = 14.9, 1H, CH₂-Bzl), 5.93 (d, *J* = 5.6, 1H, HNC_{α}), 7.12-7.20 (m, 2H, Ar), 7.23 (bs, 1H, CH triazol), 7.27-7.33 (m, 5H, Ar), 7.34-7.44 (m, 3H, Ar), 7.55 (d, *J* = 7.3, 2H, Ar), 7.74 (t, *J* = 8.2, 2H, Ar).
¹³C NMR {¹H} (400 MHz; CDCl₃): δ 27.51, 47.10, 53.25, 54.35, 66.78, 119.93, 123.18, 125.10, 127.06, 127.69, 127.92, 128.80, 129.08, 134.03, 141.19, 142.33, 143.67, 155.72, 172.55.
MS (ESI): 469.1 (M+H)⁺

### Fmoc-β-(1(-4-methoxybenzyl)-1,2,3-triazol-4-yl)-Alanine 7

¹H NMR (400 MHz; CDCl₃): δ 3.28 (dd, *J* = 14.8, *J* = 6.7, 1H, C_{β}H), 3.40 (bd, *J* = 14.8, 1H, C_{β}H'), 3.74 (s, 3H, *p*-methoxy), 4.19 (t, *J* = 6.9, 1H, CH Fmoc), 4.37 (d, *J* = 6.9 Hz, 2H, CH₂ Fmoc), 4.59 (bs, 1H, C_{α}H), 5.35 (d, *J* = 14.8, 1H, CH-Bzl), 5.46 (d, *J* = 14.8, 1H, CH-Bzl), 5.95 (d, *J* = 5.4, 1H, HNC_{α}), 6.84 (d, *J* = 8.2, 2H, Ar), 7.16 (d, *J* = 8.6, 2H, Ar), 7.21 (s, 1H, CH triazol), 7.28-7.34 (m, 2H, Ar), 7.37-7.43 (m, 2H, Ar), 7.55-7.61 (m, 2H, Ar), 7.74-7.80 (m, 2H, Ar).
MS (ESI): 499.1 (M+H)⁺

### Fmoc-β-(1-(3,5-dimethoxybenzyl)-1,2,3-triazol-4-yl)-Alanine 8

¹H NMR (400 MHz; CD₃CN): δ 3.10 (dd, *J* = 14.9, *J* = 7.6, 1H, C_{β}H), 3.20 (dd, *J* = 14.9, *J* = 4.7, 1H, C_{β}H'), 3.70 (bs, 6H, m,m-dimethoxy), 4.20 (t, *J* = 7.1, 1H, CH Fmoc), 4.30 (d, *J* = 7.1, 2H, CH₂ Fmoc), 4.41-4.46 (m, 1H, C_{α}H), 5.40 (s, 2H, CH₂-Bzl), 6.38 (bs, 3H, Bzl), 7.32 (t, *J* = 7.5, 2H, Fmoc), 7.42 (dd, *J* = 7.5, *J* = 7.1, 2H, Fmoc), 7.58-7.64 (m, 3H, Fmoc + CH triazol), 7.83 (d, *J* = 7.5 , 2H, Fmoc).
MS (ESI): 529.2 (M+H)⁺

### Fmoc-β-(1-pivaloyloxymethyl)-1,2,3-triazol-4-yl)-Alanine 9

¹H NMR (400 MHz; d6-DMSO): δ 1.07 (s, 9H, ^{t}Bu), 3.00 (dd, 1H, *J* = 14.5, *J* = 9.6, C_{β}H), 3.15 (dd, 1H, *J* = 14.5, *J* = 4.6, C_{β}H'), 3.30 (s, 1H, CH Fmoc), 4.10-4.30 (m, 3H, C_{α}H + CH₂ Fmoc), 6.26 (s, 2H, CH₂ POM), 7.31 (bt, *J* = 7.6, 2H, Fmoc), 7.41 (bt, 2H, *J* = 7.4, Fmoc), 7.67 (bt, 2H, *J* = 6.2, Fmoc), 7.88 (d, 1H, *J* = 7.6 triazol), 7.96 (s, 1H, CH triazol).
¹³C NMR {¹H} (100 MHz, CDCl₃): δ 26.72, 27.47, 38.73, 47.05, 53.18, 67.06, 69.81, 119.93, 124.57, 124.89, 125.08, 127.07, 127.68, 141.20, 141.21, 142.66, 143.64, 143.73, 155.89, 172.75, 177.65.
MS (ESI): 493.1 (M+H)⁺
IR (KBr): 3411, 3129, 2966, 1738, 1725, 1529 cm⁻¹

### Ac-β-(1-benzyl)-1,2,3-triazol-5-yl)-Ala-OEt 10

¹H NMR (400 MHz; CDCl₃): δ 1.22 (t, *J* = 7.2, 3H, CH₃ ethyl), 1.96 (s, 3H, acetyl), 3.10 (dd, *J* = 15.8, *J* = 5.9, 1H, C_{β}H), 3.16 (dd, *J* = 15.8, *J* = 6.2, 1H, C_{β}H'), 4.12-4.24 (m, 2H, CH₂ ethyl), 4.73 (ddd, *J* = 7.1, *J* = 6.2, *J* = 5.9, 1H, HC_{α}), 5.46 (d, *J* = 16.0, 1H, CH-Bzl), 5.60 (d, *J* = 16.0, 1H, CH'-Bzl), 6.38 (d, *J* = 7.1, 1H, NH), 7.12-7.20 (m, 2H, Bzl), 7.30-7.38 (m, 3H, Bzl), 7.43 (s, 1H, CH triazol)
¹³C NMR {¹H} (100 MHz, CDCl₃): δ 14.00, 22.95, 25.72, 51.28, 51.65, 51.18, 127.15, 128.37, 128.97, 132.20, 133.36, 134.70, 170.05, 170.51.
MS (ESI): 317.2 (M+H)⁺

### Ac-β-(1-(3,5-dimethoxybenzyl)-1,2,3-triazol-5-yl)-Ala-OEt 11

¹H NMR (400 MHz; CDCl₃): δ 1.21 (t, *J* = 7.1, 3H, CH₃ ethyl), 1.96 (s, 3H, acetyl), 3.08 (dd, *J* = 15.8, *J* = 5.5, 1H, C_{β}H), 3.15 (dd, *J* = 15.8 Hz, *J* = 6.4 Hz, 1H, C_{β}H'), 3.71 (s, 6H, *m*,*m*-dimethoxy), 4.13 (qd, *J* = 9.6, *J* = 7.1, 1H, CH ethyl), 4.18 (qd, *J* = 9.6, *J* = 7.1, 1H, CH ethyl), 4.73 (ddd, *J* = 7.1, *J* = 6.4, *J* = 5.5, 1H, C_{α}H), 5.38 (d, *J* = 15.6, 1H, CH-Bzl), 5.51 (d, *J* = 15.6, 1H, CH-Bzl), 6.27 (d, *J* = 2.1, 2H, Bzl), 6.35 (t, *J* = 2.1, 1H, Bzl), 6.59 (d, *J* = 7.1, 1H, NH), 7.41 (s, 1H, CH triazol).
MS (ESI): 377.1 (M+H)⁺

### Example 2: removal of protecting groups

As expected the hydrogenolysis of benzyl, p-methoxybenzyl and dimethoxybenzyl derivatives turned out to be very difficult. By using harsh conditions we managed to remove the protecting groups on compounds **2** and **10** but the same protocols applied to compounds bearing a Fmoc protecting group **(6-8)** afforded debenzylated compounds along with partial hydrogenation of the benzyl ring as well as removal of Fmoc group. Alternative, oxidising conditions using CAN were not satisfactory. Some difficulties where encountered with the selective deprotection of the P2 protecting groups. The best results were obtained using the protecting group described by Sharpless, i.e. azidomethyl pivalate (POM-N₃). Noteworthy is that this protecting group has been recently used in a very elegant synthesis of α-CF₃-substitued azahistidine.

This protecting group is orthogonal when used with BOC and it is possible to remove POM and Fmoc in a "one pot" synthesis if needed.

### A. Selective removal of Benzyl protecting group:

### • affording Ac-β-(1,2,3-triazol-S-yl)-Ala-OEt

30 mg (95 µmol) of Ac-β-(1-benzyl)-1,2,3-triazol-5-yl)-Ala-OEt **10** were introduced in 1.5 mL of MeOH into a one-neck round-bottom flask. 101.3 mg (95 µmol, 1 eq.) of palladium 10 % on charcoal were added and the mixture was put under H₂ atmosphere and stirred at R.T. during 72 h. The crude mixture was then filtered through celite and a Millipore HVLP membrane, to yield to 22.9 mg of product. The yield was quantitative.

### • affording Boc-β-(1,2,3-triazol-4-yl)-Alanine

In a pressure tank, 5 mg (14.4 µmol) of Boc-β-(1-benzyl)-1,2,3-triazol-4-yl)-alanine **2** were introduced to 3.84 mg (3.6 µmol, 0.25 eq.) of Pearlman catalyst *i.e*. Pd(OH)₂ on charcoal (20 % pure, 50 % water in weight) in 2 mL of MeOH. The tank was flushed with N₂ for 5 min and then 10 bars of H₂ were admitted. The reaction was stirred at 45 °C over 72 h.
The crude mixture was clarified on a syringe Millipore separation filter and analysed by LC/MS, revealing a peak of expected m/z accounting for more than 90 % of the total UV signal area

### B. Selective removal of either POM or Boc protecting groups:

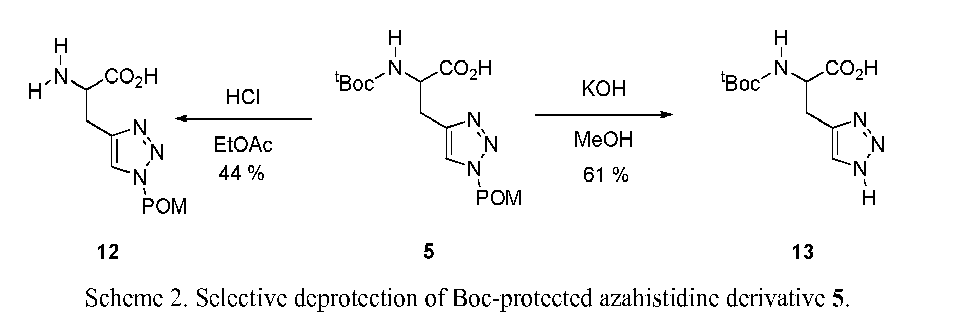

At this stage, selective deprotection of the ^{t}Boc group on compound **5** was achieved using HCl in ethyl acetate to furnish **12.** In parallel, selective removal of the pivaloyl group was cleanly achieved using 1M NaOH in MeOH without affecting the ^{t}Boc group to afford compound **13** (Scheme 2).
We checked the potential racemization of the azahistidine compounds under the deprotection conditions. Treatment of compound **5** (5 mg) overnight with NaOD/D₂O (100 µL of a 40% solution) in CD₃OD (400 µL) resulted in removal of the POM group without incorporation of deuterium at the alpha position (checked by ¹H NMR).

### Selective removal of BOC protecting group, affording H-β-((1-pivaloyloxymethyl)-1,2,3-triazol-4-yl)-Alanine 12

2.2 mL of HCl 35 % were introduced on 100 mg (0.27 mmol) of Boc-β-(1-pivaloyloxymethyl)-1,2,3-triazol-4-yl)-Ala-OH **5** in 7.8 mL of ethyl acetate. Under stirring at R.T. the reacting mixture, initially biphasic, turned rapidly to a homogenous yellowish liquid. After 3 h, the mixture is diluted in water and washed three times with dichloromethane. The aqueous phase was then freeze dried to obtain 94 mg of a yellowish solid.
The crude product is purified by preparative HPLC using a water/acetonitrile gradient with formic acid as ion pairing agent. 32.2 mg of pure white solid are obtained with a yield of 44 %.

### Selective removal of POM protecting group, affording Boc-β-(1, 2, 3-triazol-4-yl)-Alanine 13

150 mg (0.40 mmol) of Boc-β-(1-(pivaloyloxymethyl)-1,2,3-triazol-4-yl)-Ala-OH **5** were added to 2.1 mL of methanol with 2.5 mL of NaOH 1M (2.5 mmol, 6.2 eq.). The mixture was stirred for one day at R.T., then acidified to pH 3 and extracted with ethyl acetate. The organic fractions were pooled and dried with MgSO₄. After evaporation 92.6 mg of amorphous solid were obtained with a yield of 61 %.

### C. Selective removal of either Fmoc protecting group in the presence of POM:

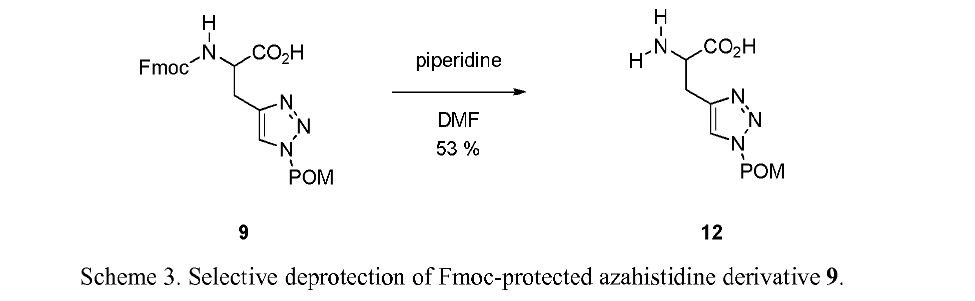

In a similar fashion, when compound **9** was treated under classical conditions for removal of the Fmoc protecting group (piperidine), we obtained compound **12** (Scheme 3). This last result should permit the use of compound **9** for insertion of azahistidine derivatives in peptides using standard SPPS strategies.

### Selective removal of Fmoc protecting group, affording H-β-((1-pivaloyloxymethyl)-1,2,3-triazol-4-yl)-Alanine 12

99 mg (0,20 mmol) of Fmoc-β-(1-pivaloyloxymethyl)-1,2,3-triazol-4-yl)-Ala-OH **9** were introduced in 5 mL of DMF with 13.5 µl (0.22 mmol, 1.1 eq.) of piperidine. The mixture was stirred at R.T. for 3 h 45 min and evaporated to obtain a white solid. The crude product was then triturated with diethyl ether to remove the dibenzofulvene-piperidine adduct. After drying, 29.3 mg (53 %) of purified solid were obtained.

### Analytical data:

### Ac-β-(1,2,3-triazol-5-yl)-Ala-OEt from 10

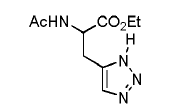

¹H NMR (400 MHz; CDCl₃): δ 1.19 (t, *J* = 7.0, 3H, CH₃ ethyl), 1.89 (s, 3H, acetyl), 3.10-3.30 (m, 2H, C_{β}HH'), 4.13 (q, *J* = 7.0, 2H, CH₂ ethyl), 4.86 (bs, 1H, C_{α}H), 7.18 (bs, 1H, C_{α}NH), 7.41 (s, 1H, CH triazol), 7.75 (bs, NH triazol).

### H-β-(1-(pivaloyloxymethyl)-1,2,3-triazol-4-yl)-Alanine 12

¹H NMR (400 MHz; D₂O): δ 1.26 (s, 9H, ^{t}Bu), 3.30 (dd, 1H, *J* = 15.8, *J* = 6.8, C_{β}H), 3.36 (dd, 1H, *J* = 15.8, *J* = 5.2, C_{β}H), 4.06 (dd, 1H, *J* = 6.8, *J* = 5.2, C_{α}H), 6.33 (s, 2H, CH₂POM), 8.06 (s, 1H, CH triazol).
¹³C NMR {¹H} (100 MHz, D₂O): δ 25.81, 26.16, 38.41, 54.16, 71.22, 125.42, 142.14, 172.91, 179.74.
MS (ESI): 271.1 (M+H)⁺
IR (KBr): 3142, 3044, 2969, 2591, 2088, 1740, 1617, 1586 cm⁻¹

### Boc-β-(1,2,3-triazol-4-yl)-Alanine 13

¹H NMR (400 MHz; CD₃OD): δ 1.37 (s, 9H, ^{t}Bu), 3.06 (dd, 1H, *J* = 14.9, *J* = 8.7, C_{β}H), 3.24 (dd, 1H, *J* = 14.9, *J* = 4.6, C_{β}H), 4.38 (dd, *J* = 8.7, *J* = 4.6, C_{α}H), 7.57 (s, 1H, CH triazol).
¹³C NMR {¹H} (100 MHz, d6-DMSO): δ 26.72, 28.15, 53.39, 78.19, 131.5, 142.8, 155.30, 173.08
MS (ESI): 257.1
IR (KBr): 3159, 3981, 2602, 2360, 1694, 1520 cm-

### Example 3: the GHK analogue

As an example, compound **9** was used for the solid phase synthesis of a Gly-His-Lys analogue, an endogenous tripeptide known as a growth-modulating factor, as a strong activator of wound healing and as a copper chelator. The synthesis was performed on a chlorotrityl resin using Fmoc-glycine, compound **9** (D enantiomer) and Fmoc-D-lysine(Boc). After cleavage of the peptide from the resin using TFA, the two remaining protecting groups (*id est* Fmoc on the glycine and POM on the azahistidine side chain) were removed using NaOH in MeOH. After trituration in cold ether, GHK analogue **14** was obtained as a single isomer.

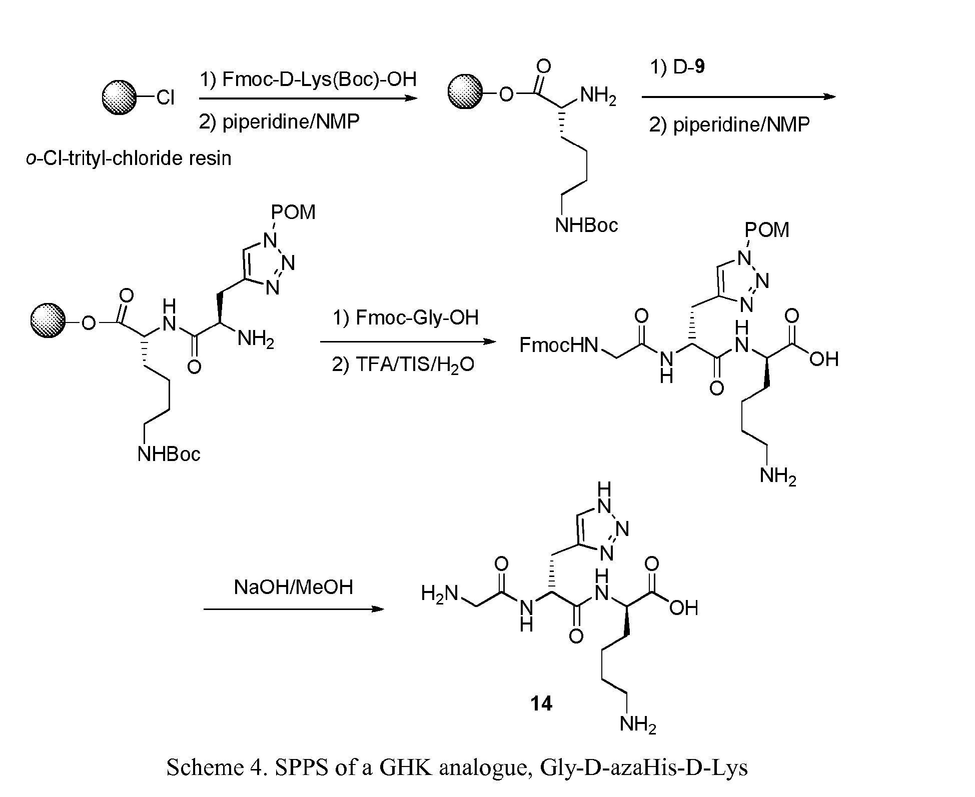

### Synthesis of Gly-D-azaHis-D-Lys 14

The synthesis of the tripeptide was achieved according to a literature protocol using DIC and HOBt as coupling reagents. Fmoc-Lys(Mtt)-OH and Fmoc-His(Mtt)-OH were replaced by Fmoc-D-Lys(Boc)-OH and Fmoc-D-azaHis(POM)-OH, respectively. Cleavage from the resin was performed using 10 mL of a cocktail composed of TFA/TIS/H₂O (9.5/0.3/0.2) for 4 hours at room temperature. After filtration, TFA was removed by evaporation, the resulting solid was dissolved in water and the solution was lyophilized. 100 mg of the peptide TFA salt was then subjected to deprotection (Fmoc and POM) by addition of 650 µL of a 1 M NaOH aqueous solution (2.2 eq.) in 3.5 mL of MeOH for 5 hours at room temperature. After addition of HCl (1M) to reach pH 4, the solution was evaporated and the solid was taken up in MeOH. NaCl was filtrated and the resulting solution was evaporated. The resulting solid was triturated in cold diethyl ether yielding peptide **14** (yield 36 %) as a white solid with a purity of 90% (estimated by LC/MS and ¹H NMR). Configuration of the compound 14 was checked by HPLC and ¹H NMR. As previously mentionned, no epimerisation of the azahistidine moiety was noticed, and no epimerisation of the lysine moiety was noticed.

### Analytical Data:

¹H NMR (400 MHz; D₂O): δ 1.19-1.31 (m, 2H, CH₂ δ Lys), 1.50-1.80 (m, 4H, CH₂ β and γ Lys), 2.88 (t, 2H, *J* = 7.2 CH₂ ε Lys), 3.13 (dd, 1H, *J* = 7.7, *J* = 15.2, CH₂ β azaHis), 3.20 (dd, 1H, *J* = 6.2, *J* = 15.2, CH₂ β azaHis), 3.74 (AB system, 2H, *J* = 16.1, CH₂ Gly), 4.13 (dd, 1H, *J* = 5.4, *J* = 7.5, CH α Lys), 4.51-4.82 (m, 1H superimposed with water, CH α azaHis), 7.68 (s, 1H, CH triazol).
¹³C NMR {¹H} (100 MHz, D₂O): δ 21.84, 26.14, 26.69, 30.36, 39.08, 40.24, 53.26, 53.84, 127.29, 140.06, 166.8, 171.58, 176.73.
HRMS (ES-) calcd for C₁₃H₂₂N₇O₄ : 340.1733; found : 340.1723.

### Material and Methods

### Chemicals

Amino acid derivatives were purchased from Bachem, Fluka and Acros Organics. Reagents and solvents for synthesis and RP-HPLC were from Sigma, Fluka or Normapur products and used without further purification. Aryl-azide derivatives were prepared by standard protocol.

### Eguipment

### LC/MS analysis

*HPLC:* Waters system (2525 binary gradient module, in-line degasser, 2767 sample manager, 2996 Photodiode Array Detector), eluents: A: 99.9% water / 0.1% HCOOH; B: 99.9% ACN / 0.1% HCOOH
analytical RP-HPLC column: X-bridge C18 column (100x4.6 mm, 3.5 µm particle size, 135 Å pore size), 1 mL/min flow rate, 20 µL sample is injected
preparative RP-HPLC column: X-bridge C18 column (150x19 mm, 5 µm particle size, 135 Å pore size), 17 mL/min flow rate
using a 8 or 25 min gradient of 95% A/5% B to 100% B for analysis and purification respectively.
*MS:* Waters Micromass ZQ system (electrospray ionization), ZQ2000 quadrupole analyser, Mass Lynx 4.0 software, source temperature 120°C, cone voltage 20V, continuous sample injection at 0.3 mL/min flow rate, mass spectra were recorded in positive ion mode in the m/z 100-1000 range.

### NMR analysis

¹H- and ¹³C-NMR spectra were recorded at room temperature at a frequency of 400 MHz and 100 MHz respectively using a Bruker Avance 400 Ultrashield. Samples were dissolved in 400 µL of deuterated solvent. Chemical shifts are given in ppm and the coupling constants in Hz.

## Claims

1. Compounds of formula (Ia) and (Ib) : 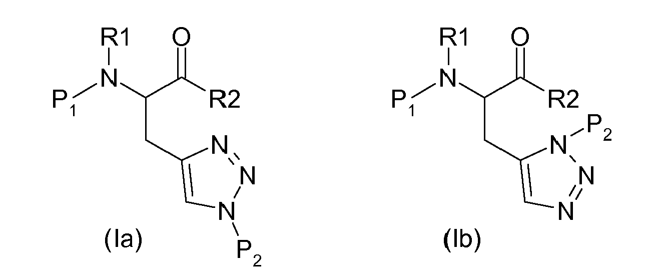 wherein
R1 is chosen between hydrogen, C₁-C₆alkyl; aryl-C₁-C₆alkyl;
R2 is chosen between hydroxy, C₁-C₆alkyloxy, aryl-C₁-C₆alkyloxy;
P1 and P2 are either identical or different nitrogen protecting groups;
P1 and P2 are chosen between Fmoc, POM, ethoxycarbonyl, Boc, Bn, Bz, CBz, allyl, Alloc, Ac, formyl, DMB, PMB or all other protecting groups used in organic chemistry.

2. A compound according to claim 1, wherein the protecting groups P₁ and P₂ are orthogonal and chosen between Fmoc, POM, ethoxycarbonyl, Boc, Bn, Bz, CBz, allyl, Alloc, Ac, formyl, DMB, PMB or all other protecting groups used in organic chemistry.

3. A compound according to claim 1 or 2, wherein the protecting group P₁ is either Fmoc or Boc.

4. A compound according to any of one of the precedent claims, wherein the protecting group P₂ is chosen between POM, ethoxycarbonyl, Bn, Bz, CBz, allyl, Alloc, Ac, formyl, DMB, PMB or all other protecting groups used in organic chemistry.

5. A compound (Ia) according to any one of the precedent claims, wherein R1 is hydrogen, R2 is hydroxy, the protecting group P₁ is chosen between Fmoc and Boc, and the protective group P₂ is POM.

6. A compound according to any one of the precedent claims, wherein the compound is an unnatural amino acid of the L series.

7. A compound according to any one of the precedent claims, wherein the compound is an unnatural amino acid of the D series.

8. A method to prepare a compound of any one of the precedent claims using the copper catalysed version of Huisgen cycloaddition comprising:
a) the use of a catalyst chosen between: Cu(OAc)₂ and sodium ascorbate, Cu/C, CuI/DiPEA, CuI(PPh₃), CuBr(Et₃N), CuBr(2,6-lutidine), copper found in zeolites or CuSO₄ and sodium ascorbate, Cp^{*}RuCl(PPh₃)₂, Cp*RuCl(COD) or Cp*RuCl(NBD);
b) the use of a solvent which can be chosen from any one of the group consisting of: water, tert-butanol, acetonitrile, dimethylformamide (DMF), benzene, toluene or a mixture thereof.

9. Use of a compound according to any one of the claims 1 to 7 in organic synthesis, either in liquid phase or on solid phase.

10. Use according to claim 9, **characterised in that** the organic synthesis is a peptide synthesis.

11. Use according to claim 9 or 10, **characterised in that** the synthesis implicates the removal of the protecting groups P₁ and/or P₂.

12. Use according to claim 11, **characterised in that** the removal of the protecting groups P₁ and/or P₂ is achieved by using soft conditions, in particular NaOH 1M in MeOH or KOH 1M in MeOH.

13. Use according to any one of the claims 9 to 12, for enabling to obtain a compound of the following formula:
